Europäisches Patentamt

European Patent Office

Office européen des brevets

(19)

(11) Publication number: **0 146 602**
**B1**

# EUROPEAN PATENT SPECIFICATION

(12)

(45) Date of publication of patent specification: **15.03.89**

(51) Int. Cl.⁴: **A 61 F 7/00, A 61 L 15/00**

(21) Application number: **84902359.3**

(22) Date of filing: **07.06.84**

(86) International application number:
**PCT/GB84/00198**

(87) International publication number:
**WO 84/04883 20.12.84 Gazette 84/30**

(54) **COOLANT DEVICE.**

(30) Priority: **08.06.83 GB 8315787**

(43) Date of publication of application:
**03.07.85 Bulletin 85/27**

(45) Publication of the grant of the patent:
**15.03.89 Bulletin 89/11**

(84) Designated Contracting States:
**AT BE CH DE FR GB LI LU NL SE**

(56) References cited:
**CH-A- 542 914**
**DE-A-2 214 306**
**DE-A-2 807 929**
**GB-A-1 306 508**
**GB-A-2 004 746**
**US-A-3 079 299**
**US-A-3 301 252**
**US-A-3 419 506**
**US-A-3 658 984**
**US-A-3 880 158**
**US-A-4 190 643**
**US-A-4 309 990**

(73) Proprietor: **BRIGGS, Joseph Hodgson**
**The Roost Abbott's Well Road Frogham**
**Near Fordingbridge Hampshire SP6 2JA (GB)**
(73) Proprietor: **TURNER, Stanley Berry**
**Lithend House Free Street**
**Bishops Waltham Hampshire (GB)**

(72) Inventor: **BRIGGS, Joseph Hodgson**
**The Roost Abbott's Well Road Frogham**
**Near Fordingbridge Hampshire SP6 2JA (GB)**
Inventor: **TURNER, Stanley Berry**
**Lithend House Free Street**
**Bishops Waltham Hampshire (GB)**

(74) Representative: **De Minvielle-Devaux, Ian**
**Benedict Peter et al**
**CARPMAELS & RANSFORD 43, Bloomsbury**
**Square**
**London WC1A 2RA (GB)**

Courier Press, Leamington Spa, England.

## Description

This invention relates to a coolant device, especially for use in the emergency treatment of burns, and in the treatment of other soft tissue injuries, such as bruises or strains of muscles, ligaments, and tendons.

It is well known that both pain and tissue damage (and, in particular, oedema) at a burn site can be substantially reduced if the burn site is cooled rapidly to a temperature which is below 30°C, and then maintained at that temperature for a substantial period of time. For example, it is suggested in a article by J. W. L. Davies in Burns, Vol. 9 (1982) pages 1 to 6, that burns should preferably be cooled in water at 20 to 25°C for an hour or more. The selection of this temperature range is said to avoid the potential hazards of frostbite or hypothermia induced by temperatures near 0°C.

It is also known that pain and tissue damage in the case of bruises, sprains and the like can be reduced by cooling the site of the injury. For example, it has been proposed in Japanese Patent Specification No. 54886606 to provide cooling in such cases by means of a coolant aerosol comprising one or more organic chemical compounds with a molecular weight greater than 80, a latent heat of evaporation of 100 cal/g or less at 25°C and a boiling point of 20 to 100°C, together with a compressed gas as a propellant. Such an aerosol is said to provide rapid cooling down to −20°C or −30°C at 5 cm from the spray nozzle, and −10°C to −25°C at 10 cm from the spray nozzle. Refrigerants such as R-12 (dichlorodifluoromethane), which have a boiling point below 20°C, are said to be unsatisfactory by reason of providing only slight cooling of the object which is being sprayed, despite considerable cooling around the nozzle of the aerosol.

The aerosol of Japanese Patent Specification No. 54-86606 suffers from the disadvantage that it is difficult to obtain a cooling effect which is of sufficient duration to give optimal pain relief and minimum tissue damage. A relatively long cooling effect is only obtained by choosing refrigerants which yield very low temperatures such as the temperatures mentioned above. This involves the risk of frostbite or hypothermia, especially to patients who are in a state of shock, such as burns patients. The aerosol disclosed in Japanese Patent Specification No. 54-86606 is therefore believed to be wholly unsuitable for the emergency treatment of burns.

An aerosol device for the treatment of cuts, burns and the like is also disclosed in US—A—3 419 506. The device delivers a composition comprising a highly viscous solution-suspension of a film-forming vinyl acetate polymer or alkyl acrylate polymer and from 10 to 50% by weight of a finely divided filler. The composition may optionally comprise a small proportion of paraffin wax. The filler is said to aid in the escape of propellant gases, resulting in a rapidly-drying foam.

We have now devised a means for cooling the site of a soft tissue injury, which avoids the above described disadvantages and which is eminently suitable for use in the emergency treatment of burns, as well as sprains and the like.

According to the present invention, there is provided a device for delivering a refrigerant foam, said device comprising a vessel containing a topically acceptable refrigerant under pressure and a topically acceptable foam-forming material consisting of a paraffin wax or a mixture of a paraffin wax and a topically acceptable oil, and means for delivering from said device an intimate mixture of the foam-forming material and the refrigerant, the foam-forming material constituting from 5 to 30% by weight of the mixture, and the boiling point of the refrigerant being such that on delivery from the device the refrigerant serves as a foaming agent for the foam-forming material.

Also provided by the invention is a foam-forming mixture for use in the treatment of soft-tissue injuries, said mixture comprising a topically acceptable refrigerant and a topically acceptable foam-forming material consisting of a paraffin wax of a mixture of paraffin wax and a topically acceptable oil, said foam-forming material constituting from 5 to 30% by weight of the mixture.

A refrigerant foam, as provided by the invention, produces a very rapid cooling of any surface with which it comes into contact. Most importantly, however, it also has a low thermal conductivity. This means that prolonged cooling is obtained without the need to resort to very low initial temperatures. For example, the device of the present invention may produce a foam which will provide cooling down to, say, 0°C, and maintain that temperature for from 15 to 30 minutes.

The prolonged cooling effect ensures that maximum pain relief and minimum tissue damage is obtained, while the avoidance of very low initial temperatures substantially eliminates the danger of frostbite or hypothermia.

The refrigerant used in the device of the invention preferably comprises one or more halogenated hydrocarbons. Particularly preferred are fluorinated hydrocarbons.

If a single refrigerant is to be used, it should preferably have a boiling point below 10°C. It is greatly preferred, however, that two or more refrigerants be used, each having different boiling points. This has the advantage that rapid evaporation of the lower boiling component generates the foam and produces the initial cooling effect, while subsequent slower evaporation of the higher boiling component or components serves to keep the foam at its low temperature. Thus, for example, the refrigerant may comprise a component having a boiling point in the range −60°C to 10°C, and more preferably in the range −40°C to −20°C, and a component having a boiling point in the range 10°C to 40°C, for example 20°C to 30°C. In particularly preferred embodiments, three refrigerants are used, namely a low boiling component having a boiling point in the range

−40°C to −20°C, for example −30°C, a high boiling component having a boiling point in the range 10°C to 40°C, for example 24°C, and an intermediate component having a boiling point in the range −20°C to 10°C, for example 4°C.

When two refrigerants are used, the lower boiling component is preferably present in an amount of from 40 to 80% by weight of the foam-forming mixture, and more preferably from 50 to 70% by weight, for example 60% by weight. The higher boiling component is advantageously present in an amount of from 20 to 40% by weight of the foam-forming mixture, and preferably from 25 to 30% by weight, for example 30% by weight.

When three refrigerants are used, the lowest boiling component is advantageously present in an amount of from 20 to 50% by weight of the foam-forming mixture, and preferably in an amount of from 30 to 40% by weight, for example 35% by weight. The high boiling component is preferably present in an amount of from 20 to 40% by weight of the foam-forming mixture, and more preferably in an amount of from 25 to 35% by weight, for example 30% by weight. The intermediate boiling component is preferably present in an amount of from 15 to 30% by weight of the foam-forming mixture, and more preferably from 20 to 25% by weight, for example 23% by weight.

The refrigerant (or the lowest boiling component thereof if a mixture of refrigerants is used) preferably has a latent heat of evaporation of at least 5000 kJ/mole, and more preferably at least 8000 kJ/mole, for example 9000 kJ/mole.

Preferred refrigerants are dichlorodifluoromethane (R12), which has a boiling point of −29.8°C, trichloromonofluoromethane (R11), which has a boiling of 23.8°C, and dichlorotetrafluoroethane (R114), which has a boiling point of 4.1°C.

The refrigerant can be used as the propellant for the device. That is to say, it is not necessary to use a compressed gas such as carbon dioxide or nitrogen, because the refrigerant has a sufficiently high vapour pressure at room temperature to drive the mixture of refrigerant and foam-forming material from the device. While a compressed gas may be used if desired, its use has the disadvantage of adding to the expense of the device, and also means that pressure is steadily lost from the device during use.

The foam-forming material is present in an amount of from 5 to 30% by weight of the foam-forming mixture, and preferably in an amount of from 10 to 20% by weight.

The foam-forming material comprises or consists of paraffin wax, which has a high specific heat, usually in the region of 0.5 cal/g. This means that the foam warms up only slowly on exposure to ambient temperatures.

The refrigerant must obviously be capable of intimate mixing with the foam-forming material, so that a foam results from evaporation of the refrigerant. Paraffin wax has the advantage of being soluble in most of the usual refrigerants, so that such intimate mixtures are easily obtained.

We have found that a wax-based refrigerant foam has very little tendency to adhere to tissue to which it is applied. In some cases, such as in the treatment of burns, this can be a great advantage, in that relatively little trauma is involved in removing such a wax-based foam from a burn site to which it has been applied. On the other hand, there may be circumstances (such as in the treatment of sprains in human patients), in which greater adhesiveness will be desired. In such a case, it may be desirable to apply an adhesion promoting substance to the tissue either before, or simultaneously with applying the refrigerant foam. Examples of suitable adhesion promoting substances are glycerine, and topically acceptable oils.

A particularly convenient additive for increasing the adhesion of a wax-based refrigerant foam is petroleum jelly. A mixture of petroleum jelly and paraffin wax as the foam-forming material has the additional advantage of greater flexibility as compared with a foam formed without petroleum jelly.

The device of the present invention may contain other additives, such as antibacterial agents. A mixture of such agents may be used to achieve a broad spectrum of antibacterial activity. An example of such a mixture is a mixture of neomycin sulphate, polymyxin sulphate and bacitracin zinc.

For certain applications, it may also be desirable to incorporate a local anaesthetic in the refrigerant foam. When the foam is to be used in the emergency treatment of burns, however, it is advisable to avoid the incorporation of an anaesthetic, because pain at a burn site is a useful indication that insufficient cooling has been achieved. This is because peripheral nerves are generally only active above 8°C.

The device of the present invention is not only useful for the treatment of human patients, but also has numerous veterinary applications, for example in the treatment of soft tissue injuries of horses and other domestic animals. In such veterinary applications, the use of an additive to promote adhesion of the refrigerant foam to the tissue being treated will generally not be necessary, because the foam will form in intimate association with the animal's hair, which thereby serves to keep the foam in place.

The device of the invention may be a conventional aerosol can containing a mixture of the foam-forming material and refrigerant. It will be appreciated, however, that other forms may be used. For example, the refrigerant and the foam-forming material may be kept in separate reservoirs, being mixed only immediately prior to release from the nozzle of the device.

The device may also be arranged to deliver first a spray of an adhesion-promoting substance, followed by the refrigerant foam.

A coolant device according to the present invention is now described, by way of example, with reference to the accompanying drawings, in which:—

Figure 1 is a schematic vertical section through an aerosol device according to the invention, and

Figure 2 is a vertical section through an alternative embodiment.

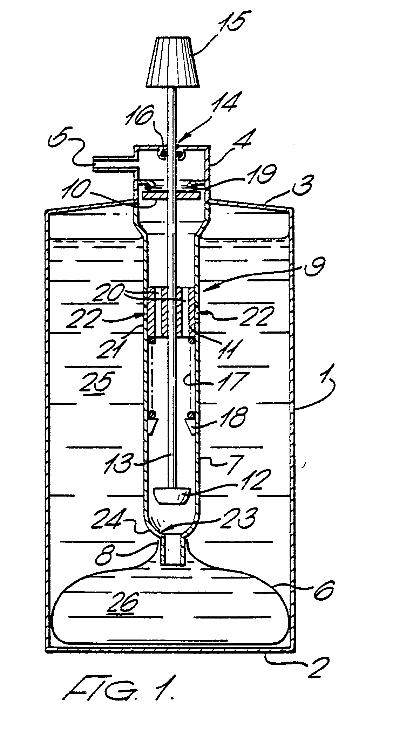

Referring to Figure 1, an aerosol device comprises a pressure-resistant metal container, formed from a generally cylindrical body 1, a base 2, and a cover 3. The container may be formed in one piece, or the body 1, the base 2, and the cover 3 may be formed separately and then assembled to form the container. A raised central portion 4 of the cover 3 forms a nozzle having an outlet 5.

The container is divided into two compartments by means of an impervious flexible bag 6 located within the container, adjacent the base 2. During operation of the device, the space enclosed by the bag 6 communicates with the outlet 5 *via* a tubular element 7, which extends axially within the body 1. The tubular element 7 is sealingly connected at one end to a neck 8 formed in the flexible bag 6, and at the other end to the cover 3 around the periphery of the raised central portion 4.

Communication of the interior of the container with the outlet 5 is controlled by means of a valve arrangement shown generally at 9. The valve arrangement comprises first, second and third valve members 10, 11 and 12 spaced a short distance apart on a rod 13 which is axially disposed in the tubular element 7. The rod 13 passes through a central aperture 14 in the raised portion 4 of the cover 3, to terminate in an actuating knob 15. A sealing ring 16 ensures that the contents of the container do not escape through the aperture 14 when the rod 13 is moved axially.

A coil spring 17 is arranged axially in the tubular element 7 between an abutment 18 and the second valve member 11. The first valve member 10 is thus biased against a seal 19 arranged in the raised portion 4 of the cover 3, to isolate the interior of the raised portion from the remainder of the interior of the container.

The second valve member 11 is in the form of a piston having channels 20 extending axially therethrough. The second valve member 11 forms a sliding fit in a cylindrical region 21 of the tubular element 7, the wall of the tubular element in this region being pierced by vents 22. The vents 22 are closed by the second valve member 11 when the first valve member 10 bears against the seal 19.

The third valve member 12 is arranged to engage against an annular rim 23, formed by a constriction 24 in the tubular element 7, when the rod 13 is fully depressed against the bias of the coil spring 17.

The device, when ready for use, contains in the main body thereof, a foam-forming mixture 25 of a topically acceptable refrigerant and a topically acceptable foam-forming material. The refrigerant has a sufficiently high vapour pressure at ambient temperatures that the contents of the device are under a pressure of a few atmospheres.

The bag 6 contains an adhesion promoting agent 26 such as glycerine. The adhesion promoting agent is also under pressure, because the bag 6 is flexible.

The operation of the device is as follows:

Partial depression of the actuating knob 15 causes the first valve member 10 to disengage from the seal 19. The vents 22, however, remain sealed by the second valve member 11, and the annular rim 23 is not yet engaged by the third valve member 12. The pressure on the bag 6 therefore causes the adhesion promoting agent 26 to rise up the tubular element 7 and be dispensed from the outlet 5 in a fine spray.

Further depression of the actuating knob 15 causes the third valve element 12 to form a seal with the angular rim 23, thereby cutting off the supply of adhesion promoting agent. Simultaneously, the second valve member 11 moves to a position in which the vents 22 are no longer closed. The foam-forming mixture 25 therefore passes through the vents 22 and thence out through the outlet 5.

On release from the device, the refrigerant component of the foam-forming mixture boils, causing the foam-forming mixture to expand to form a foam.

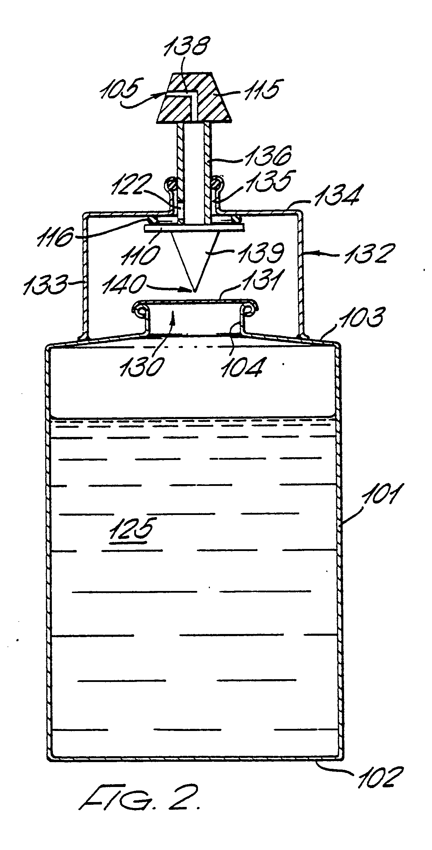

Figure 2 shows a simplified device which does not deliver a preliminary spray of adhesion promoting agent. However, it includes a modification which assists in increasing the shelf-life of the device.

The device of Figure 2 comprises a pressure-resistant container formed from a generally cylindrical body 101, a base 102 and a cover 103. The cover 103 has a centrally disposed raised portion 104 which defines an opening 130. Prior to use, the container is filled with a mixture 125 of a topically acceptable refrigerant and a topically acceptable foam-forming material. The opening 130 is then sealed with an impermeable disc 131 of frangible material such as a plastics film or metal foil.

Sealingly surrounding the raised portion 104 is a cap 132 having a cylindrical side wall 133 and flat top plate 134. The top plate 134 has a centrally disposed outlet 135, through which passes a hollow tube 136, the tube 136 forming a sliding fit in an O-ring 137 mounted at the outlet 135.

Externally of the container, the tube 136 terminates in an actuating knob 115. The bore of the tube 136 communicates with a bore 138 in the actuating knob 115, leading to an outlet nozzle 105.

Internally of the container, the tube 136 has a side opening 122 and terminates in a valve member 110. The valve member 110 is biassed by biasing means (not shown, for reasons of clarity) against a sealing ring 116. The valve member 110 is provided on its underside with a spear 139, which has its point 140 adjacent the disc 131.

When the actuating knob 115 is first depressed, the spear 139 breaks the seal formed by the disc 131, so that the foam-forming mixture passes into the space defined by the cap 132. The mixture then passes into the tube 136 *via* opening 122, and thence out through the nozzle 105, whenever the actuating knob is depressed.

This construction has the advantage that reliance is not placed on the seal between the valve member and the sealing ring until after the device is first used. The generally more effective seal provided by the sealing disc means that the device has an improved shelf-life as compared with the embodiment of Figure 1.

Foam-forming mixtures for use in the device of the present invention are now illustrated in the following Examples:—

Example 1

An aerosol device such as that illustrated in Figure 2 was charged with a mixture of the following components:

| Component | % by weight |
|---|---|
| Paraffin wax | 10 |
| R12 refrigerant | 60 |
| R11 refrigerant | 30 |

Release of the mixture from the aerosol device yielded a refrigerant foam which was eminently suited for use in the emergency treatment of burns. The foam was relatively non-adherent to skin, and maintained a temperature of approximately 0°C for about 20 minutes.

Example 2

An aerosol device was charged with the following mixture:

| Component | % by weight |
|---|---|
| Paraffin wax | 8 |
| Petroleum jelly | 12 |
| Refrigerant R11 | 35 |
| Refrigerant R12 | 30 |
| Refrigerant R114 | 23 |

On release from the device, the mixture yielded a refrigerant foam which was particularly well suited to equine veterinary use. The foam had a lower melting point than that obtained with the mixture of Example 1.

This had the advantage that it was able to wet the hair of the horse to which it was applied, allowing more rapid transfer of heat from the site of the injury.

Also, the flow velocity of the foam-forming mixture in the nozzle of the aerosol device, (and therefore the frequency of the sound produced) was reduced. This had the advantage of being less disturbing to a temperamental animal, such as a race horse.

The foam produced initial cooling to approximately 8°C, which is particularly beneficial to athletic injuries.

It will be understood that the present invention has been described above purely by way of example, and modifications of detail can be made within the scope of the invention as defined by the claims.

## Claims

1. A device for delivering a refrigerant foam, said device comprising a vessel containing a topically acceptable refrigerant under pressure and a topically acceptable foam-forming material consisting of a paraffin wax or a mixture of a paraffin wax and a topically acceptable oil, and means for delivering from said device an intimate mixture of the foam-forming material and the refrigerant, the foam-forming material constituting from 5 to 30% by weight of the mixture, and the boiling point of the refrigerant being such that on delivery from the device the refrigerant serves as a foaming agent for the foam-forming material.

2. A device according to claim 1, wherein the refrigerant comprises one or more fluorinated hydrocarbons.

3. A device according to claim 1 or claim 2, wherein the refrigerant comprises a low boiling component having a boiling point in the range −60°C to 10°C, and a high boiling component having a boiling point in the range 10°C to 40°C.

4. A device according to claim 3 wherein the low boiling component constitutes from 40 to 80% by weight of the foam-forming mixture, and the high boiling component constitutes from 10 to 40% by weight of said mixture.

5. A device according to claim 1 or claim 2, wherein the refrigerant comprises a low boiling component having a boiling point in the range −40°C to −20°C, a high boiling component having a boiling point in the range 10°C to 40°C, and an intermediate boiling component having a boiling point in the range −20°C to 10°C.

6. A device according to claim 5 wherein the low boiling component constitutes from 20 to 50% by weight of the foam-forming mixture, the high boiling component constitutes from 20 to 40% by weight of said mixture, and the intermediate boiling component constitutes from 15 to 30% by weight of said mixture.

7. A device according to any of claims 3 to 6 wherein the low boiling component is dichlorodifluoromethane, the high boiling component is trichloromonofluoromethane, and the intermediate boiling component, if present, is dichlorotetrafluoroethane.

8. A device according to any preceding claim wherein the foam-forming material constitutes from 10 to 20% by weight of the foam-forming mixture.

9. A device according to any preceding claim, wherein the foam-forming material is a paraffin wax.

10. A device according to any of claims 1 to 8 wherein the foam-forming material is a mixture of a paraffin wax and petroleum jelly.

11. A device according to any preceding claim, wherein said device being arranged to deliver first an adhesion promoting agent, and then said foam-forming mixture.

12. A foam-forming mixture for use in the treatment of soft-tissue injuries, said mixture comprising a topically acceptable refrigerant and a topically acceptable foam-forming material consisting of a paraffin wax or a mixture of paraffin wax and a topically acceptable oil, said foam-forming material constituting from 5 to 30% by weight of the mixture.

13. A foam-forming mixture according to claim 12, wherein said refrigerant is as defined in any of claims 2 to 7.

14. A foam-forming mixture according to claim 12 or claim 13, wherein the foam-forming material is as defined in any of claims 8 to 10.

**Patentansprüche**

1. Vorrichtung zur Abgabe eines Kältemittelschaumes, wobei die Vorrichtung einen Behälter mit einem topisch verträglichen unter Druck stehenden Kältemittel und ein topisch verträgliches schäumbildendes Mittel aus einem Paraffinwachs oder einer Mischung aus einem Paraffinwachs und einem topisch verträglichen Öl und eine Einrichtung zum Abgeben einer innigen Mischung aus dem schaumbildenden Material und dem Kältemittel aus der Vorrichtung aufweist, wobei das schaumbildende Material 5 bis 30 Gew.-% de Mischung bildet und der Siedepunkt des Kältemittels derart ist, daß das Kältemittel bei der Abgabe aus der Vorrichtung als Schaumbildner für das schaumbildende Material dient.

2. Vorrichtung nach Anspruch 1, wobei das kältemittel einen oder mehrere fluorinierte Kohlenwasserstoffe aufweist.

3. Vorrichtung nach Anspruch 1 oder 2, wobei das Kältemittel eine niedrig siedende Komponente mit einem Siedepunkt im Bereich von −60°C bis 10°C und eine hochsiedende Komponente mit einem Siedepunkt im Bereich von 10°C bis 40°C aufweist.

4. Vorrichtung nach Anspruch 3, wobei die niedrig siedende Komponente 40 bis 80 Gew.-% der schaumbildenden Mischung und die hochsiedende Komponente 10 bis 40 Gew.-% der Mischung bildet.

5. Vorrichtung nach Anspruch 1 oder 2, wobei das Kältemittel eine niedrig siedende Komponente mit einem Siedepunkt im Bereich von −40°C bis −20°C, eine hochsiedende Komponente mit einem Siedepunkt im Bereich von 10°C bis 40°C und eine dazwischen siedende Komponente mit einem Siedepunkt im Bereich von −20°C bis 10°C aufweist.

6. Vorrichtung nach Anspruch 5, wobei die niedrig siedende Komponente 20 bis 50 Gew.-% der schaumbildenden Mischung, die hochsiedende Komponente 20 bis 40 Gew.-% der Mischung und die dazwischen siedende Komponente 15 bis 30 Gew.-% der Mischung bildet.

7. Vorrichtung nach einem der Ansprüche 3 bis 6, wobei die niedrig siedende Komponente Dichlordifluormethan, die hochsiedende Komponente Trichlormonofluormethan und die dazwischen siedende Komponente, falls vorhanden, Dichlortetrafluorethan ist.

8. Vorrichtung nach einem der vorhergehenden Ansprüche, wobei das schaumbildende Material 10 bis 20 Gew.-% der schaumbildenden Mischung bildet.

9. Vorrichtung nach einem der vorhergehenden Ansprüche, wobei das schaumbildende Material ein Paraffinwachs ist.

10. Vorrichtung nach einem der Ansprüche 1 bis 8, wobei das schaumbildende Material ein Mischung aus einem Paraffinwachs und Petroljelly ist.

11. Vorrichtung nach einem der vorhergehenden Ansprüche, wobei die Vorrichtung ausgebildet ist, um zuerst ein Adhesionsverbesserungsmittel und dann die schaumbildende Mischung abzugeben.

12. Schaumbildende Mischung zur Verwendung bei der Behandlung von Verletzungen von Weichteilgewebe, wobei die Mischung ein topisch verträgliches Kältemittel und ein topisch verträgliches schaumbildendes Material bestehend aus einem Paraffinwachs oder aus einer Mischung aus Paraffinwachs und einem topisch verträglichen Öl aufweist, und wobei das schaumbildende Material 5 bis 30 Gew.-% der Mischung bildet.

13. Schaumbildende Mischung nach Anspruch 12, mit einem Kältemittel nach einem der Ansprüche 2 bis 7.

14. Schaumbildende Mischung nach Anspruch 12 oder 13, mit einem schaumbildenden Material nach einem der Ansprüche 8 bis 10.

**Revendications**

1. Dispositif pour délivrer une mousse réfirgrérante, ledit dispositif comprenant un récipient contenant un réfrigérant sous pression acceptable par voie topique et une matière formant mousse acceptable par voie topique consistant en une cire de paraffine ou un mélange d'une cire de paraffine et une huile acceptable par voie topique et des moyens pour délivrer à partir dudit dispositif un mélange intime de la matière formant mousse et du réfrigérant, la matière formant mousse représentant de 5 à 30% en poids du mélange et le point d'ébullition du réfrigérant étant tel que lors de la sortie du dispositif, le réfrigérant sert d'agent moussant pour la matière formant mousse.

2. Dispositif selon la revendication 1 dans lequel le réfrigérant comprend un ou plusieurs hydrocarbures fluorés.

3. Dispositif selon l'une des revendications 1 ou 2 dans lequel le réfrigérant comprend un composant à bas point d'ébullition ayant un point d'ébullition dans la gamme de −60°C à 10°C et un composant à point d'ébulittion élevée ayant un point d'ébullition dans la gamme de 10°C à 40°C.

4. Dispositif selon l revendication 3, dans lequel le composant à bas point d'ébullition représente de 40 à 80% en poids du mélange formant la mousse et le component à point d'ébullition élevé représente de 10 à 40% en poids dudit mélange.

5. Dispositif selon l'une des revendications 1 ou 2, dans lequel le réfrigérant comprend un composant à bas point d'ébullition ayant un point d'ébullition dans la gamme de −40°C à −20°C, un composant à point d'ébullition élevé ayant un point d'ébullition dans la gamme de 10°C à 40°C et un composant à point d'ébullition intermédiaire ayant un point d'ébullition compris dans la gamme de −20°C à 10°C.

6. Dispositif selon la revendication 5 dans lequel le composant à bas point d'ébullition représente de 20 à 50% en poids du mélange formant mousse, le composant à point d'ébullition élevé représente de 20 à 40% en poids dudit mélange et le composant à point d'ébullition intermédiaire représente de 15 à 30% en poids dudit mélange.

7. Dispositif selon l'une quelconque des revendications 3 à 6, dans lequel le composant à bas point d'ébullition est le dichlorodifluorométhane, le composant à point d'ébullition élevé est le trichloromonofluorométhane et le composant à point d'ébullition intermédiaire, s'il est présent, est le dichloro-tétrafluoroéthane.

8. Dispositif selon l'une quelconque des revendications 1 à 7, dans lequel la matière formant mousse représente de 10 à 20% en poids du mélange formant mousse.

9. Dispositif selon l'une quelconque des revendications 1 à 8, dans lequel la matière formant mousse est de la cire de paraffine.

10. Dispositif selon l'une quelconque des revendications 1 à 8 dans lequel la matière formant mousse est un mélange d'une cire de paraffine et de gelée de pétrole.

11. Dispositif selon l'une quelconque des revendications 1 à 10, dans lequel le dispositif est agencé pour délivrer tout d'abord un agent favorisant l'adhésion puis le mélange formant mousse.

12. Un mélange formant mousse destiné à être utilisé dans le traitement de blessures des tissus, ledit mélange comprenant un réfrigérant acceptable par voie topique et une matière formant mousse acceptable par voie topique consistant en une cire de paraffine ou un mélange de cire de paraffine et d'une huile acceptable par voie topique, ladite matière formant mousse représentant de 5 à 30% en poids du mélange.

13. Mélange formant mousse selon la revendication 12, dans lequel le réfrigérant est tel que défini dans l'une quelconque des revendications 2 à 7.

14. Mélange formant mousse selon l'une des revendications 12 ou 13, dans lequel la matière formant mousse est telle que définie dans l'une quelconque des revendications 8 à 10.

FIG. 1.

*FIG. 2.*